# EUROPEAN PATENT SPECIFICATION

(11) **EP 4 081 227 B1**
(45) Date of publication and mention of the grant of the patent: **01.04.2026**
(21) Application number: 20905004.6
(22) Date of filing: 22.12.2020
(51) Int. Cl.: A61P 29/00, A61K 35/33, A61K 38/20

(54) **SUPPRESSION OF INTERLEUKIN-17 PRODUCTION AND INHIBITION OF TH17 CELL GENERATION BY FIBROBLASTS AND PRODUCTS THEREOF**
UNTERDRÜCKUNG DER INTERLEUKIN-17-PRODUKTION UND HEMMUNG DER TH17-ZELLERZEUGUNG DURCH FIBROBLASTEN UND PRODUKTE DAVON
SUPPRESSION DE LA PRODUCTION D'INTERLEUKINE-17 ET INHIBITION DE LA GÉNÉRATION DE CELLULES TH17 PAR DES FIBROBLASTES ET LEURS PRODUITS

(30) Priority: 26.12.2019 US 201962953843 P
(43) Date of publication of application: 02.11.2022
(73) Proprietor: SpinalCyte LLC, Houston, TX 77289 (US)
(72) Inventor: ICHIM, Thomas, Houston, Texas 77058 (US); O'HEERON, Pete, Houston, Texas 77059 (US)
(74) Representative: J A Kemp LLP
(86) International application number: PCT/US2020/066591
(87) International publication number: WO 2021/133793

(56) References cited:
- WO-A1-2018/091698
- WO-A1-2019/108756
- WO-A1-2019/213518
- WO-A1-2020/243504
- WO-A1-2021/097434
- US-A1- 2011 236 427
- US-A1- 2014 348 806
- US-A1- 2016 256 496
- JANKOWSKI ET AL.: "Anti-inflammatory effect of oxytocin in rat myocardial infarction", BASIC RESEARCH IN CARDIOLOGY, vol. 105, no. 2, March 2010 (2010-03-01), pages 205 - 218, XP019780567

## Description

### TECHNICAL FIELD

Described herein are fibroblasts for use in a method of treating inflammation in an individual.

### BACKGROUND

Interleukin-17 was described in an early publication as a cDNA cloned from a CD4+ T cell library with a predicted 155-amino acids sequence that contains an N-terminal signal peptide and exhibits 72% amino acid identity with HVS13, an open reading frame from a T-lymphotropic Herpesvirus saimiri, and 63% with murine CTLA8. High levels of human interleukin-17 (hIL-17_ were induced from primary peripheral blood CD4+ T cells upon stimulation. When expressed in CV1/EBNA cells, recombinant hIL-17 was secreted in both glycosylated and nonglycosylated forms. A hIL-17.Fc fusion protein and supernatants from cells transfected with hIL-17 induced IL-6 and IL-8 production and enhanced the surface expression of the intracellular adhesion molecule-1 (ICAM-1) in human fibroblasts [1]. Early characterization of the effects of IL-17 included studies assessing its impact on hematopoiesis. In one such study investigators found that although devoid of direct effects on cells of hematopoietic origin, hIL-17 and the product of its viral counterpart, ORF13, stimulate epithelial, endothelial, and fibroblastic cells to secrete cytokines such as IL-6, IL-8, and granulocyte-colony-stimulating factor, as well as prostaglandin E2. Furthermore, when cultured in the presence of hIL-17, fibroblasts could sustain the proliferation of CD34+ hematopoietic progenitors and their preferential maturation into neutrophils. These observations suggested that hIL-17 may constitute (a) an early initiator of the T cell-dependent inflammatory reaction; and (b) an element of the cytokine network that bridges the immune system to hematopoiesis [2].

The IL-17 family comprises IL17A, IL-17B, IL-17C, IL-17D, IL-17E and IL-17F. IL-17E is also known as IL-25. All members of the IL-17 family have a similar protein structure. Their protein sequences contain four highly conserved cysteine residues. These conserved cysteine residues are critical to the correct 3-dimensional shape of the entire protein molecule. The members of the IL-17 family do not exhibit a significant sequence homology with other cytokines. Among IL-17 family members, the IL-17F isoforms 1 and 2 (ML-1) have the highest sequence homology with IL-17A (55 and 40%, respectively). They follow by IL-17B, which has 29% similarity to IL-17A, IL-17D (25%), IL-17C (23%), and IL-17E (17%). In mammals, the sequences of these cytokines are highly conserved. For instance, the sequence homology between the corresponding human and mouse proteins is usually between 62-88% [7].

US 2014/348806 A1 relates to a cell-based therapy for the pulmonary system. US 2016/256496 A1 relates to a pharmaceutical composition and method for the treatment of orthopedic pathologies. WO 2018/091698 A1 relates to compositions for the treatment of immune-related diseases. WO 2020/243504 A1 relates to concurrent activation of regenerative and tolerogenic processes by fibroblast-based compositions for the treatment of multiple sclerosis. WO 2019/213518 A1 relates to pain-reducing effects of fibroblasts and treatment of pain. WO 2021/097434 A1 relates to fibroblast therapy for inflammatory bowel disease. WO 2019/108756 A1 relates to interaction of fibroblasts and immune cells for activation and uses thereof. Jankowski et al. (Basic Res Cardiol. vol. 105, March 2010, pages 205-218) relates to anti-inflammatory effects of oxytocin in rat myocardial infarction. US 2011/236427 A1 relates to antagonists of NR2F6 for augmenting the immune response.

### SUMMARY OF THE INVENTION

The invention provides fibroblasts for use in a method of treating inflammation in an individual, the method comprising the step of providing to the individual an effective amount of said fibroblasts, wherein said fibroblasts express interleukin-3 receptor, CD73 and CD56.

The invention is defined in the appended claims. Any aspects, embodiments and examples of the present disclosure which do not fall under the scope of the appended claims do not form part of the invention and are merely provided for illustrative purposes.

References to the methods of treatment by therapy or surgery or in vivo diagnosis methods in the description are to be interpreted as references to compounds, pharmaceutical compositions and medicaments of the present invention for use in those methods.

### SUMMARY OF THE TECHNICAL TEACHINGS

Described herein are compositions and methods to treat or reduce or prevent inflammation in individuals, including from inflammatory cytokines (that also may be called proinflammatory cytokines), such as from any type of immune cells (such as helper T cells and macrophages, for example) that promote inflammation.

Described herein are methods and compositions related to reducing deleterious effects of inflammatory cytokines, including excess inflammatory cytokines. In particular aspects, the methods and compositions provide treatment or prevention of inflammation directly or indirectly related to inflammatory cytokines, including at least IL17. The methods and compositions in specific aspects concern fibroblasts and/or derivatives thereof that have the ability to inhibit cells that produce IL17. The fibroblasts may be of any kind. In cases wherein derivatives of fibroblasts are utilized, the derivative may be of any kind, and in any case the fibroblasts and/or derivatives may or may not have one or more particular characteristics, such as one or more particular markers. In specific aspects, the fibroblasts are activated, such as to suppress IL-17 production, and in specific cases the fibroblasts are activated with oxytocin, such as in a range of from 0.1 µM to 10 µM and that may be for a duration of exposure from 1 minute to 96 hours. The range of oxytocin concentration may be from 0.1-10, 0.1-5, 0.1-2, 0.1-1, 0.5-10, 0.5-5, 0.5-2, 0.5-1, 1-10, 1-5, 1-2, 2-10, 2-5, or 5-10 µM. The duration of exposure may be from 1 minute-96 hours, 1 minute-72 hours, 1 minute-48 hours, 1 minute-24 hours, 1 minute-12 hours, 1 minute-1 hour, 1-96, 1-72, 1-48, 1-23, 1-12, 12-96, 12-72, 12-48, 12-24, 24-96, 24-72, 24-48, 24-36, 36-96, 36-72, 36-48, 48-96, 48-72, or 72-96 hours.

Methods are described herein for treatment or prevention of inflammation of any kind, including directly or indirectly related to excessive, chronic production of one or more inflammatory cytokines. Examples of medical conditions include at least inflammatory diseases, neurological diseases, autoimmune diseases, and so forth. Specific medical conditions that may be treated or prevented include at least atherosclerosis, cancer, Graft-versus-host disease (GvHD), cystic fibrosis, depression, rheumatoid arthritis, psoriasis, multiple sclerosis, inflammatory bowel diseases, rheumatoid arthritis (RA), multiple sclerosis (MS), psoriasis, Crohn's disease, systemic lupus erythematosus (SLE), asthma, Behçet's disease, hyper IgE syndrome, ankylosing spondylitis, scleroderma, polymyositis, dermatomyositis, inclusion body myositis, idiopathic inflammatory myopathies such as immune-mediated necrotizing myopathy. An autoimmune disease of the blood system may be treated or prevented, such as aplastic anemia or idiopathic thrombocytopenic purpura. An autoimmune disease of the digestive system may be treated or prevented, such as Crohn's disease or ulcerative colitis. An autoimmune disease of the nervous system may be treated or prevented, such as multiple sclerosis or myasthenia gravis. An autoimmune disease of the visual system may be treated or prevented, such as one or two or more selected from the group consisting of uveitis, keratitis, and Sjogren's syndrome. An autoimmune disease of the vascular system may be treated or prevented, such as Behcet's disease or Wegener's granulomatosis. An autoimmune disease of the epidermal system may be treated or prevented, such as one or two or more selected from the group consisting of psoriasis, pemphigus, Stevens-Johnson syndrome, and vitiligo. An autoimmune disease of the respiratory system may be treated or prevented, such as chronic obstructive pulmonary disease or interstitial pneumonia. An autoimmune disease of the endocrine system may be treated or prevented, such as one or two or more selected from the group consisting of type 1 diabetes, autoimmune thyroiditis, Graves' disease, and Hashimoto's thyroiditis.

Aspects of the disclosure include methods of inhibiting one or more inflammatory cytokines in an individual, comprising the step of providing to the individual an effective amount of fibroblasts and/or functional derivatives thereof that reduce production of IL-17 from IL17-producing cells, that reduce cellular response to IL17, and/or that reduce generation of Th17 cells. In some aspects, the fibroblasts express CD73, interleukin-3 receptor, and/or CD56.

In some cases, prior to the providing step the fibroblasts are exposed to one or more agents and/or conditions that activate NF-kappa B in the fibroblasts. The NF-kappa B-activated fibroblasts may produce one or more of IL-10, IL-35, and IL37, in specific aspects. In some cases, the one or more agents and/or conditions are selected from the group consisting of hydrogen peroxide, ozone, TNF-alpha, IL-1, osmotic shock, mechanical agitation, and a combination thereof. The IL17-producing cells may or may not be immunological cells, such as those selected from the group consisting of a) monocytes; b) T cells; c) Th17 cells; d) neutrophils; e) stromal cells; f) mesenchymal stem cells; g) dendritic cells; and h) a combination thereof. In particular aspects, the providing step comprises contacting the fibroblasts and/or functional derivatives thereof (for example, apoptotic bodies, microvesicles, lysates, exosomes, or a combination thereof) with IL17-producing cells *in vitro* or *in vivo.* In specific aspects, the fibroblast derivative is an apoptotic body that comprises phosphatidylserine on the membrane. The fibroblast derivative may be an exosome that expresses one or more protein markers selected from the group consisting of CD9, CD63, CD81, and a combination thereof. In certain aspects, the fibroblasts and/or fibroblast derivatives thereof are provided to the individual intravenously, subcutaneously, intravenously, intramuscularly, subcutaneously, intradermally, locally, by inhalation, orally, topically, epidurally, by injection, rectally, and/or by catheter.

Individuals subject to methods described by the disclosure may have inflammation, including inflammation associated with an inflammatory disease, neurological disease, or autoimmune disease. In specific aspects, the individual has atherosclerosis, cancer, Graft-versus-host disease (GvHD), cystic fibrosis, depression, rheumatoid arthritis, psoriasis, multiple sclerosis, inflammatory bowel diseases, rheumatoid arthritis (RA), multiple sclerosis (MS), psoriasis, Crohn's disease, systemic lupus erythematosus (SLE), asthma, Behçet's disease, hyper IgE syndrome, or a combination thereof.

Aspects of the disclosure include methods of treating inflammation in an individual, comprising the step of providing to the individual an effective amount of fibroblasts and/or functional derivatives thereof that reduce production of IL-17 from IL17-producing cells, that reduce cellular response to IL17, and/or that reduce generation of Th17 cells. In some cases, prior to the providing step the fibroblasts are exposed to one or more agents and/or conditions that activate NF-kappa B in the fibroblasts. NF-kappa B-activated fibroblasts may produce one or more of IL-10, IL-35, and IL37. In specific aspects, the one or more agents and/or conditions are selected from the group consisting of hydrogen peroxide, ozone, TNF-alpha, IL-1, osmotic shock, mechanical agitation, and a combination thereof. IL17-producing cells may or may not be immunological cells, such as immunological cells are selected from the group consisting of a) monocytes; b) T cells; c) Th17 cells; d) neutrophils; e) stromal cells; f) mesenchymal stem cells; g) dendritic cells; and h) a combination thereof. In some cases, the providing step comprises contacting the fibroblasts and/or functional derivatives thereof (apoptotic bodies, microvesicles, lysates, exosomes, or a combination thereof, for example) with IL17-producing cells *in vitro* or *in vivo.* In certain cases, the fibroblast derivative is an apoptotic body that comprises phosphatidylserine on the membrane. The fibroblast derivative may be an exosome that expresses one or more protein markers selected from the group consisting of CD9, CD63, CD81, and a combination thereof. In certain aspects, the fibroblasts and/or fibroblast derivatives thereof are provided to the individual intravenously, subcutaneously, intravenously, intramuscularly, subcutaneously, intradermally, locally, by inhalation, orally, topically, epidurally, by injection, rectally, and/or by catheter. The individual may have inflammation associated with an inflammatory disease, neurological disease, or autoimmune disease. The individual may have atherosclerosis, cancer, Graft-versus-host disease (GvHD), cystic fibrosis, depression, rheumatoid arthritis, psoriasis, multiple sclerosis, inflammatory bowel diseases, rheumatoid arthritis (RA), multiple sclerosis (MS), psoriasis, Crohn's disease, systemic lupus erythematosus (SLE), asthma, Behçet's disease, hyper IgE syndrome, or a combination thereof.

### BRIEF DESCRIPTION OF THE DRAWING

For a more complete understanding of the present disclosure, reference is now made to the following descriptions taken in conjunction with the accompanying drawing, in which:

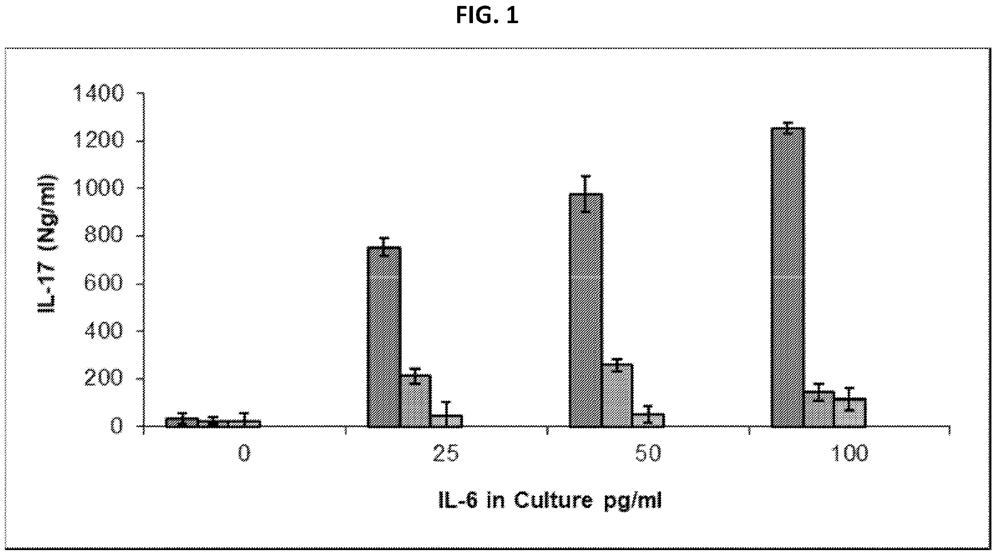

FIG. 1 shows *in vitro* suppression of IL-17 production in CD4 cells primed by IL-6 by culture with fibroblasts activated by oxytocin; bars indicate (from left to right): no fibroblasts, 10,000 fibroblasts, and 20,000 fibroblasts. IL-17 production in the CD4 cell culture increased with increasing IL-6 concentration and decreased with increasing fibroblast concentration.
FIG. 2 shows *in vivo* suppression of IL-17 production in serum of mice with collagen-induced arthritis (CIA) produced by injection with collagen as described in [3] using treatment with, from left to right, non-CIA saline control, CIA saline control, CIA plus bone marrow mesenchymal stem cells (MSCs), and CIA plus CD73-positive fibroblasts.

### DETAILED DESCRIPTION

### I. Examples of Definitions

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein and that different aspects may be combined.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." It is specifically contemplated that x, y, or z may be specifically excluded from an aspect.

Throughout this application, the term "about" is used according to its plain and ordinary meaning in the area of cell and molecular biology to indicate that a value includes the standard deviation of error for the device or method being employed to determine the value.

The term "comprising," which is synonymous with "including," "containing," or "characterized by," is inclusive or open-ended and does not exclude additional, unrecited elements or method steps. The phrase "consisting of" excludes any element, step, or ingredient not specified. The phrase "consisting essentially of" limits the scope of described subject matter to the specified materials or steps and those that do not materially affect its basic and novel characteristics. It is contemplated that aspects described in the context of the term "comprising" may also be implemented in the context of the term "consisting of" or "consisting essentially of."

A variety of aspects of this disclosure can be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 *etc.,* as well as individual numbers within that range, for example, 1, 2, 3, 4, 5, and 6. This applies regardless of the breadth of the range. When ranges are present, the ranges may include the range endpoints.

"Express" and "expression" refer to the process by which information (*e.g*., genetic and/or epigenetic information) is converted into the structures present in a cell or secreted therefrom. Accordingly, as used herein, "expression" may refer to transcription, translation, or polynucleotide and/or polypeptide modifications (*e.g*., posttranslational modification of a polypeptide).

"Pharmaceutical composition" as used herein refers to any composition that comprises one or more therapeutically or biologically active agents such as cells, exosomes, apoptotic bodies, and/or conditioned media thereof.

The term "subject," as used herein, may be used interchangeably with the term "individual" and generally refers to an individual in need of a therapy. The subject can be a mammal, such as a human, dog, cat, horse, pig or rodent. The subject can be a patient, *e.g.,* have or be suspected of having or at risk for having a disease or medical condition, including for inflammation, for example. For subjects having or suspected of having a medical condition directly or indirectly associated with inflammation, the medical condition may be of one or more types. The subject may have a disease or be suspected of having the disease. The subject may be asymptomatic. The subject may be of any gender. The subject may be of a certain age, such as at least 30, 35, 40, 45, 50, 55, 60, 65, 70, 75, 80, 85, 90, 95, or 100 or more.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, "treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific aspects, treatment refers to the lessening in severity or extent of at least one symptom and may alternatively or in addition refer to a delay in the onset of at least one symptom.

The term "extracellular vesicle" as used herein is a particle naturally released from virtually every cell type in the body that is surrounded by a phospholipid bilayer and cannot replicate. Extracellular vesicles contain distinct lipids, proteins, sugars, adhesion integrins, growth factors, receptors, cytokines, protease inhibitors, and nucleic acids that reflect their cells of origin. Extracellular vesicles include exosomes (generally ranging from 30-150 nm), microvesicles (generally ranging from 30 nm-2µm), and apoptotic bodies (500 nm-2µm).

The term "conditioned media" as used herein is the spent media harvested from cultured cells. It contains metabolites, growth factors, and/or extracellular matrix proteins secreted into the medium by the cultured cells. Examples may include metabolites such as glucose, amino acids, and nucleosides; growth factors such as interleukins, EGF (epidermal growth factor), and PDGF (platelet-derived growth factor); and matrix proteins such as collagen, fibronectin, and various proteoglycans. Fibroblasts are extremely heterogeneous multi-functional cells that play a role in wound healing, developmental processes, and tumor development. Fibroblasts are capable of producing and releasing into the culture media various immune modulators including peptide growth factors, cytokines, chemokines and inflammatory mediators.

### II. Uses of Fibroblasts and Derivatives Thereof

Described herein are means, methods and compositions of matter useful for suppression of inflammation associated with IL-17 production and/or generation of Th17 cells. Described herein is the use of fibroblasts in an unmodified and/or modified state to (1) suppress cellular production of IL-17, (2) to suppress responsiveness of cells to IL-17, and to (3) suppress generation of Th17 cells. In one aspect of the disclosure, products derived from fibroblasts (that also may be referred to as fibroblast derivatives, such as fibroblast apoptotic bodies, fibroblast lysates, fibroblast microvesicles, and/or fibroblast-derived exosomes and/or conditioned media) are utilized for suppression of IL-17 production, suppression of IL-17 responsiveness, and/or suppression of Th17 cell differentiation.

Aspects of the disclosure include methods of suppressing production of IL-17 from one or more cells and/or types of cells of any kind by contacting the cell(s) with one or more fibroblasts and/or fibroblast derivatives of any kind. Aspects of the disclosure include methods of suppressing production of IL-17 from a plurality of cells of any kind by contacting the plurality of cells of any kind with a plurality of fibroblasts of any kind. Although the IL-17-producing cells may be of any kind, in specific aspects, the IL-17-producing cells are immunological cells. Although the IL17-producing immunological cells may be of any kind, in specific aspects the immunological cells are selected from the group consisting of: a) monocytes; b) T cells; c) Th17 cells; d) neutrophils; e) stromal cells; f) mesenchymal stem cells; g) dendritic cells; h) NK cells; i) NKT cells; and j) a combination thereof. The IL-17 may be of any kind of the family, including with respect to one or more of the following members of the IL-17 family: a) IL17A; b) IL-17B; c) IL-17C; d) IL-17D; e) IL-17E; f) IL-17F; and g) a combination thereof. The fibroblast(s) and/or fibroblast derivatives may be in contact with the IL-17 producing cell(s) *in vitro* and/or *in vivo.* The fibroblasts and/or fibroblast derivatives may contact the IL-17-producing cells in a manner and/or under conditions to stimulate engagement of one or more adhesion molecules. Adhesion molecules found on IL-17 producing cells include: a) ICAM; b) VCAM; c) CTLA-4; d) CD80; e) CD86; and f) VLA-4.

In specific aspects, contact between the fibroblasts and the IL-17-producing cells occurs by means of one or more particles or compositions of any kind derived from the fibroblasts that then contact the IL-17-producing cells. In specific cases, fibroblast-derived particle from the fibroblasts include apoptotic bodies, microvesicles, exosomes, or a combination thereof. In specific cases, the apoptotic bodies express or produce one or more particular proteins, peptides, carbohydrates, lipids, phospholipids, or other compounds on their membrane. In one specific example, the apoptotic bodies produce phosphatidylserine on their membrane. In another example, calreticulin is expressed on the surface of exosomes additionally or alternatively to phosphatidylserine. In particular cases, the exosomes from the fibroblasts express one or more protein markers on their membrane, for example markers selected from the group consisting of a) CD9; b) CD63; c) CD81; and d) a combination thereof.

In particular aspects, the fibroblasts are from a group of tissues selected from the group consisting of a) placenta; b) cord blood; c) mobilized peripheral blood; d) omentum; e) hair follicle; f) skin; g) bone marrow; h) adipose tissue; i) Wharton's Jelly; and j) a combination thereof. In cases wherein the fibroblasts are derived from mobilized peripheral blood, the peripheral blood mobilization in specific aspects refers to blood extracted from an individual who has received one or more treatments that promotes entrance of fibroblasts into circulation. In specific aspects, mobilization of fibroblasts and/or fibroblast progenitors into circulation is accomplished by administration of one or more agents to the individual, before the blood is drawn, such as agents selected from the group consisting of: a) VLA-5 antibodies; b) G-CSF; c) M-CSF; d) GM-CSF; e) FLT-3L; f) TNF-alpha; g) EGF; h) FGF-1; i) FGF-2; j) FGF-5; k) VEGF; and 1) a combination thereof.

In specific aspects, the fibroblasts are exposed to one or more agents that activate the fibroblasts. In specific aspects, to suppress IL-17 production the fibroblasts are activated with an effective amount of oxytocin.

In particular aspects, the fibroblasts are treated with one or more agents capable of activating NF-kappa B. The activation of NF-kappa B may or may not be transient. In specific cases, the activation of NF-kappa B endows the fibroblasts with an ability to inhibit mixed lymphocyte reaction. In specific aspects, a mixed lymphocyte reaction entails mixing populations of lymphocytes together and measuring at least one reaction that occurs. In particular aspects, mixed lymphocyte reaction is an *ex vivo* cellular immune assay that occurs between two allogeneic lymphocyte populations (same species but genetically distinct). When the fibroblasts are treated with one or more agents capable of activating NF-kappa B, the activation of NF-kappa B endows the fibroblasts with an ability to produce IL-10, IL-35, and/or IL-37. In specific aspects, the one or more agents capable of activating NF-kappa B comprises exposure to an agent selected from the group consisting of a) hydrogen peroxide; b) ozone; c) TNF-alpha; d) interleukin-1; e) osmotic shock; f) mechanical agitation; and g) a combination thereof.

The fibroblasts may be administered to an individual by any suitable means, including intravenous, subcutaneous, intravenous, intramuscular, subcutaneous, intradermal, locally, by inhalation, orally, topically, epidurally, by injection, rectally, by catheter, and so forth.

In specific aspects, the disclosure provides means of suppressing production of the IL-17 by treatment of cells that produce IL-17 with an effective amount of fibroblasts and/or derivatives thereof. Treatment of cells that produces IL-17 with an effective amount of fibroblasts and/or fibroblast derivatives thereof may comprise (1) contact of the IL-17 producing cells with fibroblasts, and/or (2) contact of mediators produced by the fibroblasts with the IL-17-producing cells. In one aspect, mediators produced by the fibroblasts are exosomes from the fibroblasts. In another aspect, mediators are soluble cytokines, metabolites, fatty acids and/or other mediators of biological activity. In another aspect, such mediators are apoptotic bodies, intracellular vesicles, nucleic acids, and/or ribosomes. In one aspect, the IL-17-producing cells are T cells, and in other aspects the IL17-producing cells are Th17 cells. In some aspects, prior to and/or during their use fibroblasts are treated to enhance their ability to inhibit IL-17 production from IL17-producing cells.

For the practice of the methods of the disclosure, in some aspects fibroblasts may be generated through means known in the art from a variety of tissues. In specific aspects, following obtaining fibroblasts, and prior to their use, activation of NF-kappa B in the fibroblasts is performed to enhance the ability of the fibroblasts to suppress IL-17 production from the IL17-producing cells.

### III.Fibroblasts, Fibroblasts Derivatives, and Manipulation and/or Production Thereof

In particular aspects, fibroblasts are obtained or manipulated prior to administering them and/or derivatives thereof to an individual in need. The amount of any types of cells for administration to an individual may depend on the type of disease to be treated, of the severity and stage of the disease, and/or of the type of cells or cellular products to be injected for the treatment. The cells may be prepared for administration in a pharmaceutically acceptable carrier, for example a sterile saline isotonic solution. In some aspects, the pharmaceutically acceptable carrier may comprise one or more additional agents, such as FAS ligand, IL-2R, IL-1 Ra, IL-2, IL-4, IL-8, IL-10, IL-20, IL-35, HLA-G, PD-L1, I-309, IDO, iNOS, CD200, Galectin 3, sCR1, arginase, PGE-2, aspirin, atorvastatin, fluvastatin, lovastatin, pravastatin, rosuvastatin, simvastatin, pitavastatin, n-acetylcysteine, rapamycin, IVIG, naltrexone, TGF-beta, VEGF, PDGF, CTLA-4, anti-CD45RB antibody, hydroxychloroquine, leflunomide, auranofin, dicyanogold, sulfasalazine, methotrexate, glucocorticoids, etanercept, adalimumab, abatacept, anakinra, certolizumab, Etanercept-szzs, golimumab, infliximab, rituximab, tocilizumab, cyclosporine, IFN-gamma, everolimus, rapamycin, VEGF, FGF-1, FGF-2, angiopoietin, HIF-1-alpha, or a combination thereof.

In one aspect of the disclosure, fibroblasts are administered to a subject by any suitable route, including by injection (such as intramuscular or intravenous injection), including in hypoxic areas. Suitable routes include intravenous, subcutaneous, intrathecal, oral, intrarectal, intrathecal, intra-omentral, intraventricular, intrahepatic, and intrarenal.

The number of administrations of cells to an individual will depend upon the factors described herein at least in part and may be optimized using routine methods in the art. In specific aspects, a single administration is required. In other aspects, a plurality of administration of cells is required. It should be appreciated that the system is subject to variables, such as the particular need of the individual, which may vary with time and circumstances, the rate of loss of the cellular activity as a result of loss of cells or activity of individual cells, and the like. Therefore, it is expected that each individual could be monitored for the proper dosage, and such practices of monitoring an individual are routine in the art.

In one aspect of the disclosure, cells (such as fibroblasts) are cultured *ex vivo* using means known in the art for preserving viability and proliferative ability of the cells. In specific aspects for fibroblasts, there may be modification of known culture techniques to achieve one or more desired effects for the cells, such as to decrease visibility of fibroblasts to a recipient immune system. In one aspect, cells (for example, fibroblasts) are cultured in conditions that lack one or more xenogeneic components, such as fetal calf serum. In specific aspects, the disclosure encompasses the substitution of fetal calf serum with human platelet rich plasma, platelet lysate, umbilical cord blood serum, autologous serum, and/or defined cytokine mixes as an additional feature, for example to reduce the immunogenicity of the cells (such as fibroblasts).

In some aspects, fibroblasts are utilized in an autologous manner. In another aspect, allogeneic fibroblasts are utilized for the practice of the methods of the disclosure. In some aspects, fibroblasts are utilized in a xenogeneic manner. Various sources of fibroblasts may be used for the practice of the methods of the disclosure, and, these include at least : a) foreskin; b) adipose tissue; c) skin; d) bone marrow; e) placenta; f) umbilical cord; g) amniotic fluid; h) umbilical cord blood; i) ear lobe skin; j) embryonic fibroblasts; k) plastic surgery related by-product; 1) nail matrix; m) heart; n) blood vessels; o) skeletal muscle; p) liver; q) pancreas; r) brain and s) a combination thereof. In specific aspects, the fibroblasts are placental, fetal, neonatal or adult or mixtures thereof.

In one aspect, the disclosure encompasses the use of activation of fibroblasts prior to therapeutic use, and/or administration of agents that act as "regenerative adjuvants" for the fibroblasts prior to and/or during their use. In specific aspects, the cells in a formulation display typical fibroblast morphologies when growing in cultured monolayers. Specifically, cells may display an elongated, fusiform or spindle appearance with slender extensions, or cells may appear as larger, flattened stellate cells that may have cytoplasmic leading edges. A mixture of these morphologies may also be observed. The cells express proteins characteristic of normal fibroblasts including a fibroblast-specific marker, CD90 (Thy-1), a 35 kDa cell-surface glycoprotein, and the extracellular matrix protein, collagen. In some aspects the fibroblast dosage formulation is an autologous cell therapy product comprised of a suspension of autologous fibroblasts, grown from a biopsy of each individual's own skin using standard tissue culture procedures. In one aspect, the fibroblasts of the disclosure can also be used to generate other cell types for tissue repair or regeneration.

The fibroblasts utilized in the methods and compositions of the disclosure may be generated, in one aspect, by outgrowth from a biopsy of the recipient's own tissue (in the case of autologous preparations) or tissue of healthy donors (for allogeneic preparations). The fibroblasts utilized in the methods and compositions of the disclosure may be generated, in one aspect, by outgrowth from a biopsy of the recipient's own skin (in the case of autologous preparations), or skin of healthy donors (for allogeneic preparations). In some aspects fibroblasts are used from donors under the age of 18. In another aspect, fibroblasts are transfected with one or more genes (or fibroblasts in a plurality have been separately transfected with one or more different genes) to allow for enhanced growth and overcoming of the Hayflick limit. Subsequent derivation of cells expansion in culture using standard cell culture techniques may occur. In specific cases, skin tissue (dermis and epidermis layers) may be biopsied from a subject's post-auricular area. In one aspect, the starting material is comprised of three 3-mm punch skin biopsies collected using standard aseptic practices. The biopsies are collected by the medical practitioner, placed into a vessel, such as a vial, comprising sterile phosphate buffered saline (PBS). The biopsies may be shipped in a 2-8° C refrigerated shipper to the manufacturing facility. In one aspect, after arrival at a manufacturing facility, the biopsy is inspected and, upon acceptance, transferred directly to a manufacturing area. In specific cases, upon initiation of the process, the biopsy tissue is then washed prior to enzymatic digestion. After washing, a Liberase Digestive Enzyme Solution is added without mincing, and the biopsy tissue is incubated at 37.0±2° C for one hour. Time of biopsy tissue digestion is a process parameter that can affect the viability and growth rate of cells in culture. Liberase is a collagenase/neutral protease enzyme cocktail obtained formulated from Lonza Walkersville, Inc. (Walkersville, Md.) and unformulated from Roche Diagnostics Corp. (Indianapolis, Ind.). Alternatively, other commercially available collagenases may be used, such as Serva Collagenase NB6 (Helidelburg, Germany). After digestion, Initiation Growth Media (IMDM, GA, 10% Fetal Bovine Serum (FBS)) is added to neutralize the enzyme, cells are pelleted by centrifugation and resuspended in 5.0 mL Initiation Growth Media. Alternatively, centrifugation is not performed, with full inactivation of the enzyme occurring by the addition of Initiation Growth Media only. Initiation Growth Media is added prior to seeding of the cell suspension into a T-175 cell culture flask for initiation of cell growth and expansion. A T-75, T-150, T-185 or T-225 flask can be used in place of the T-75 flask. Cells are incubated at 37.0±2.0° C. with 5.0±1.0% CO₂ and fed with fresh Complete Growth Media every three to five days. All feeds in the process are performed by removing half of the Complete Growth Media and replacing the same volume with fresh media. Alternatively, full feeds can be performed. Cells should not remain in the T-175 flask greater than 30 days prior to passaging. Confluence is monitored throughout the process to ensure adequate seeding densities during culture splitting. When cell confluence is greater than or equal to 40% in the T-175 flask, they are passaged by removing the spent media, washing the cells, and treating with Trypsin-EDTA to release adherent cells in the flask into the solution. Cells are then trypsinized and seeded into a T-500 flask for continued cell expansion. Alternately, one or two T-300 flasks, One Layer Cell Stack (1 CS), One Layer Cell Factory (1 CF) or a Two Layer Cell Stack (2 CS) can be used in place of the T-500 Flask. Morphology is evaluated at each passage and prior to harvest to monitor the culture purity throughout the culture purity throughout the process. Morphology is evaluated by comparing the observed sample with visual standards for morphology examination of cell cultures. The cells display typical fibroblast morphologies when growing in cultured monolayers. Cells may display either an elongated, fusiform or spindle appearance with slender extensions, or appear as larger, flattened stellate cells which may have cytoplasmic leading edges. A mixture of these morphologies may also be observed. Fibroblasts in less confluent areas can be similarly shaped, but randomly oriented. The presence of keratinocytes in cell cultures is also evaluated. Keratinocytes appear round and irregularly shaped and, at higher confluence, they appear organized in a cobblestone formation. At lower confluence, keratinocytes are observable in small colonies. Cells are incubated at 37.0±2.0° C. with 5.0± 1.0% CO₂ and passaged every three to five days in the T-500 flask and every five to seven days in the ten layer cell stack (10CS). Cells should not remain in the T-500 flask for more than 10 days prior to passaging. Quality Control (QC) release testing for safety of the Bulk Drug Substance includes sterility and endotoxin testing. When cell confluence in the T-500 flask is .gtoreq.95%, cells are passaged to a 10 CS culture vessel. Alternately, two Five Layer Cell Stacks (5 CS) or a 10 Layer Cell Factory (10 CF) can be used in place of the 10 CS. 10CS. Passage to the 10 CS is performed by removing the spent media, washing the cells, and treating with Trypsin-EDTA to release adherent cells in the flask into the solution. Cells are then transferred to the 10 CS. Additional Complete Growth Media is added to neutralize the trypsin and the cells from the T-500 flask are pipetted into a 2 L bottle containing fresh Complete Growth Media. The contents of the 2 L bottle are transferred into the 10 CS and seeded across all layers. Cells are then incubated at 37±2.0° C. with 5.0±1.0% CO₂ and fed with fresh Complete Growth Media every five to seven days. Cells should not remain in the 10CS for more than 20 days prior to passaging. In one aspect, the passaged dermal fibroblasts are rendered substantially free of immunogenic proteins present in the culture medium by incubating the expanded fibroblasts for a period of time in protein free medium, Primary Harvest When cell confluence in the 10 CS is 95% or more, cells are harvested. Harvesting is performed by removing the spent media, washing the cells, treating with Trypsin-EDTA to release adherent cells into the solution, and adding additional Complete Growth Media to neutralize the trypsin. Cells are collected by centrifugation, resuspended, and in-process QC testing performed to determine total viable cell count and cell viability.

In a particular aspect, about 50 million to 500 million fibroblast cells are administered to the subject. For example, about 50 million to about 100 million fibroblast cells, about 50 million to about 200 million fibroblast cells, about 50 million to about 300 million fibroblast cells, about 50 million to about 400 million fibroblast cells, about 100 million to about 200 million fibroblast cells, about 100 million to about 300 million fibroblast cells, about 100 million to about 400 million fibroblast cells, about 100 million to about 500 million fibroblast cells, about 200 million to about 300 million fibroblast cells, about 200 million to about 400 million fibroblast cells, about 200 million to about 500 million fibroblast cells, about 300 million to about 400 million fibroblast cells, about 300 million to about 500 million fibroblast cells, about 400 million to about 500 million fibroblast cells, about 50 million fibroblast cells, about 100 million fibroblast cells, about 150 million fibroblast cells, about 200 million fibroblast cells, about 250 million fibroblast cells, about 300 million fibroblast cells, about 350 million fibroblast cells, about 400 million fibroblast cells, about 450 million fibroblast cells or about 500 million fibroblast cells may be administered to the subject.

In some aspects, fibroblast exosomes are used to decrease IL-17 production from IL17-producing cells. Exosomes for use in the current disclosure may be purified as follows, as an example: In one aspect, fibroblasts are cultured using means known in the art for preserving viability and proliferative ability of fibroblasts. The disclosed methods and compositions may be applied both for individualized autologous exosome preparations and for exosome preparations obtained from established cell lines, for experimental or biological use. In one aspect, a method is more specifically based on the use of chromatography separation methods for preparing membrane vesicles, particularly to separate the membrane vesicles from potential biological contaminants, wherein the microvesicles are exosomes, and cells utilized for generating the exosomes are fibroblast cells.

Indeed, membrane vesicles, particularly exosomes, could be purified, and possess the desired ability. In one aspect, a strong or weak, such as strong, anion exchange may be performed. In addition, in a specific aspect, the chromatography is performed under pressure. Thus, more specifically, it may comprise high performance liquid chromatography (HPLC). Different types of supports may be used to perform the anion exchange chromatography. In specific cases, these may include cellulose, poly(styrene-divinylbenzene), agarose, dextran, acrylamide, silica, ethylene glycol-methacrylate co-polymer, or mixtures thereof, e.g., agarose-dextran mixtures. To illustrate this, it is possible to mention the different chromatography equipment composed of supports as mentioned above, particularly the following gels: SOURCE. POROS^{®}, SEPHAROSE^{®}, SEPHADEX^{®}, TRISACRYL^{®}, TSK-GEL SW OR PW^{®}, SUPERDEX^{®}TOYOPEARL HW and SEPHACRYL^{®}, for example, which are suitable for the application of methods of this disclosure. Therefore, in a specific aspect, this disclosure relates to a method of preparing membrane vesicles, particularly exosomes, from a biological sample such as a tissue culture containing fibroblasts, comprising at least one step during which the biological sample is treated by anion exchange chromatography on a support selected from cellulose, poly(styrene-divinylbenzene), silica, acrylamide, agarose, dextran, ethylene glycol-methacrylate co-polymer, alone or in mixtures, optionally functionalized.

In addition, to improve the chromatographic resolution, it is desirable to use supports in bead form. Ideally, these beads have a homogeneous and calibrated diameter, with a sufficiently high porosity to enable the penetration of the objects under chromatography (*i.e.* the exosomes). In this way, given the diameter of exosomes (generally between 50 and 100 nm), , one can use high porosity gels, particularly between 10 nm and 5 µm, more preferably between approximately 20 nm and approximately 2 µm, even more preferably between about 100 nm and about 1 µm. For the anion exchange chromatography, the support used must be functionalized using a group capable of interacting with an anionic molecule. Generally, this group is composed of an amine which may be ternary or quaternary, which defines a weak or strong anion exchanger, respectively. Within the scope of this disclosure, one can use a strong anion exchanger. In this way, a chromatography support as described above, functionalized with quaternary amines, is used. Therefore, according to a more specific aspect of the disclosure, the anion exchange chromatography is performed on a support functionalized with a quaternary amine. In some cases, this support should be selected from poly(styrene-divinylbenzene), acrylamide, agarose, dextran and silica, alone or in mixtures, and functionalized with a quaternary amine. Examples of supports functionalized with a quaternary amine include the gels SOURCEQ. MONO Q, Q SEPHAROSE^{®}, POROS^{®} HQ and POROS^{®} QE, FRACTOGEL^{®}TMAE type gels and TOYOPEARL SUPER^{®}Q gels.

A particular support to perform the anion exchange chromatography comprises poly(styrene-divinylbenzene). An example of this type of gel is SOURCE Q gel, particularly SOURCE 15 Q (Pharmacia). This support offers the advantage of very large internal pores, thus offering low resistance to the circulation of liquid through the gel, while enabling rapid diffusion of the exosomes to the functional groups, which are particularly important parameters for exosomes given their size. The biological compounds retained on the column may be eluted in different ways, particularly using the passage of a saline solution gradient of increasing concentration, *e.g*. from 0 to 2 M. A sodium chloride solution may particularly be used, in concentrations varying from 0 to 2 M, for example. The different fractions purified in this way are detected by measuring their optical density (OD) at the column outlet using a continuous spectrophotometric reading. As an indication, under the conditions used in the examples, the fractions comprising the membrane vesicles were eluted at an ionic strength comprised between approximately 350 and 700 mM, depending on the type of vesicles.

Different types of columns may be used to perform this chromatographic step, according to requirements and the volumes to be treated. For example, depending on the preparations, it is possible to use a column from approximately 100 µl up to 10 ml or greater. In this way, the supports available have a capacity which may reach 25 mg of proteins/ml, for example. For this reason, a 100 µl column has a capacity of approximately 2.5 mg of proteins which, given the samples in question, allows the treatment of culture supernatants of approximately 2 1 (which, after concentration by a factor of 10 to 20, for example, represent volumes of 100 to 200 ml per preparation). It is understood that higher volumes may also be treated, by increasing the volume of the column, for example. In addition, it is also possible to combine the anion exchange chromatography step with a gel permeation chromatography step. In this way, according to a specific aspect of the disclosure, a gel permeation chromatography step is added to the anion exchange step, either before or after the anion exchange chromatography step. In one aspect, the permeation chromatography step takes place after the anion exchange step. In addition, in a specific variant, the anion exchange chromatography step is replaced by the gel permeation chromatography step. The present disclosure demonstrates that membrane vesicles may also be purified using gel permeation liquid chromatography, particularly when this step is combined with an anion exchange chromatography or other treatment steps of the biological sample, as described in detail below.

To perform the gel permeation chromatography step, a support selected from silica, acrylamide, agarose, dextran, ethylene glycol-methacrylate co-polymer or mixtures thereof, *e.g.,* agarose-dextran mixtures, are preferably used. As an illustration, for gel permeation chromatography, a support such as SUPERDEX^{®}200HR (Pharmacia), TSK G6000 (TosoHaas) or SEPHACRYL^{®} S (Pharmacia) may be used. The process according to the disclosure may be applied to different biological samples that comprise fibroblasts. In particular, these may comprise of a biological fluid from a subject (bone marrow, peripheral blood, *etc. etc*.), a culture supernatant, a cell lysate, a pre-purified solution or any other composition comprising membrane vesicles.

In this respect, in a specific aspect of the disclosure, the biological sample is a culture supernatant of membrane vesicle-producing fibroblast cells.

In addition, according to one aspect of the disclosure, the biological sample is treated, prior to the chromatography step, to be enriched with membrane vesicles (enrichment stage). In this way, in a specific aspect, this disclosure relates to a method of preparing membrane vesicles from a biological sample, characterized in that it comprises at least: b) an enrichment step, to prepare a sample enriched with membrane vesicles, and c) a step during which the sample is treated by anion exchange chromatography and/or gel permeation chromatography.

In one aspect, the biological sample is a culture supernatant treated so as to be enriched with membrane vesicles. In particular, the biological sample may be composed of a pre-purified solution obtained from a culture supernatant of a population of membrane vesicle-producing cells or from a biological fluid, by treatments such as centrifugation, clarification, ultrafiltration, nanofiltration and/or affinity chromatography, particularly with clarification and/or ultrafiltration and/or affinity chromatography. Therefore, a particular method of preparing membrane vesicles according to this disclosure more particularly comprises the following steps: a) culturing a population of membrane vesicle (*e.g.,* exosome) producing cells under conditions enabling the release of vesicles, b) a step of enrichment of the sample in membrane vesicles, and c) an anion exchange chromatography and/or gel permeation chromatography treatment of the sample.

As indicated above, the sample (*e.g.* supernatant) enrichment step may comprise one or more centrifugation, clarification, ultrafiltration, nanofiltration and/or affinity chromatography steps on the supernatant. In a first specific aspect, the enrichment step comprises (i) the elimination of cells and/or cell debris (clarification), possibly followed by (ii) a concentration and/or affinity chromatography step. In another specific aspect, the enrichment step comprises an affinity chromatography step, optionally preceded by a step of elimination of cells and/or cell debris (clarification). A particular enrichment step comprises (i) the elimination of cells and/or cell debris (clarification), (ii) a concentration and (iii) an affinity chromatography. The cells and/or cell debris may be eliminated by centrifugation of the sample, for example, at a low speed, preferably below 1000 g, between 100 and 700 g, for example. Preferred centrifugation conditions during this step are approximately 300 g or 600 g for a period between 1 and 15 minutes, for example.

The cells and/or cell debris may also be eliminated by filtration of the sample, possibly combined with the centrifugation described above. The filtration may particularly be performed with successive filtrations using filters with a decreasing porosity. For this purpose, filters with a porosity above 0.2 µm, *e.g.* between 0.2 and 10 µm, are preferentially used. It is particularly possible to use a succession of filters with a porosity of 10 µm, 1 µm, 0.5 µm followed by 0.22 µm.

A concentration step may also be performed, in order to reduce the volumes of sample to be treated during the chromatography stages. In this way, the concentration may be obtained by centrifugation of the sample at high speeds, *e.g.* between 10,000 and 100,000 g, to cause the sedimentation of the membrane vesicles. This may consist of a series of differential centrifugations, with the last centrifugation performed at approximately 70,000 g. The membrane vesicles in the pellet obtained may be taken up with a smaller volume and in a suitable buffer for the subsequent steps of the process. The concentration step may also be performed by ultrafiltration. In fact, this ultrafiltration allows both to concentrate the supernatant and perform an initial purification of the vesicles. According to a particular aspect, the biological sample (*e.g.,* the supernatant) is subjected to an ultrafiltration, preferably a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibres (Amicon, Millipore, Sartorius, Pall, GF, Sepracor). The use of membranes with a cut-off threshold below 1000 kDa, preferably between 300 kDa and 1000 kDa, or even more preferably between 300 kDa and 500 kDa, is advantageous.

The affinity chromatography step can be performed in various ways, using different chromatographic support and material. It is advantageously a non-specific affinity chromatography, aimed at retaining (*i.e.,* binding) certain contaminants present within the solution, without retaining the objects of interest (*i.e.,* the exosomes). It is therefore a negative selection. In some cases, an affinity chromatography on a dye is used, allowing the elimination (*i.e.,* the retention) of contaminants such as proteins and enzymes, for instance albumin, kinases, dehydrogenases, clotting factors, interferons, lipoproteins, or also co-factors, *etc.* More preferably, the support used for this chromatography step is a support as used for the ion exchange chromatography, functionalized with a dye. As specific example, the dye may be selected from Blue SEPHAROSE^{®}.(Pharmacia), YELLOW 86, GREEN 5 and BROWN 10 (Sigma). The support is more preferably agarose. It should be understood that any other support and/or dye or reactive group allowing the retention (binding) of contaminants from the treated biological sample can be used in the instant disclosure.

In one aspect, a membrane vesicle preparation process within the scope of this disclosure comprises the following steps: a) the culture of a population of membrane vesicle (*e.g.* exosome) producing cells under conditions enabling the release of vesicles, b) the treatment of the culture supernatant with at least one ultrafiltration or affinity chromatography step, to produce a biological sample enriched with membrane vesicles (*e.g.* with exosomes), and c) an anion exchange chromatography and/or gel permeation chromatography treatment of the biological sample. In a preferred aspect, step b) above comprises a filtration of the culture supernatant, followed by an ultrafiltration, preferably tangential. In another preferred aspect, step b) above comprises a clarification of the culture supernatant, followed by an affinity chromatography on dye, preferably on Blue SEPHAROSE^{®}.

In addition, after step c), the material harvested may, if applicable, be subjected to one or more additional treatment and/or filtration stages d), particularly for sterilization purposes. For this filtration treatment stage, filters with a diameter less than or equal to 0.3 µm are preferentially used, or even more preferentially, less than or equal to 0.25 µm. Such filters have a diameter of 0.22 µm, for example. After step d), the material obtained is, for example, distributed into suitable devices such as bottles, tubes, bags, syringes, *etc.,* in a suitable storage medium. The purified vesicles obtained in this way may be stored cold, frozen or used extemporaneously. Therefore, a specific preparation process may comprise at least the following steps: c) an anion exchange chromatography and/or gel permeation chromatography treatment of the biological sample, and d) a filtration step, particularly sterilizing filtration, of the material harvested after stage c). In a first variant, the process may comprise: c) an anion exchange chromatography treatment of the biological sample, and d) a filtration step, particularly sterilizing filtration, on the material harvested after step c).

In another variant, the process according to the disclosure comprises: c) a gel permeation chromatography treatment of the biological sample, and d) a filtration step, particularly sterilizing filtration, on the material harvested after step c). According to a third variant, the process may comprise: c) an anionic exchange treatment of the biological sample followed or preceded by gel permeation chromatography, and d) a filtration step, particularly sterilizing filtration, on the material harvested after step c).

### IV. Gene Editing Techniques of the Disclosure

The introduction of nucleic acids into cells can be accomplished by various means. In one aspects, transduction is the infection of a target cell such as the fibroblast by a virus that promotes genetic modification of the target cell. Many viruses bind and infect mammalian cells and can be used to introduce genetic material (e.g., a donor gene, such as a gene encoding a transcription factor or other gene product) into the host cell as part of their replication cycle. In viruses modified for gene transfer, the donor gene (e.g., a gene encoding a transcription factor or other gene product) is inserted into the viral genome. Additional modifications may be made to the virus to improve infectivity or tropism (e.g., pseudotyping), to reduce or eliminate replicative competency, and/or to reduce immunogenicity. The newly-introduced donor gene will be expressed in the infected host cell or organism and, if replacing a defective host gene, can ameliorate conditions or diseases caused by the defective gene.

Numerous examples of viral vectors that can be used to deliver genetic material (e.g., a donor gene, such as a gene encoding a transcription factor or other gene product) include, but are not limited to, a retrovirus, adenovirus (e.g., Ad2, Ad5, Ad11, Ad12, Ad24, Ad26, Ad34, Ad35, Ad40, Ad48, Ad49, Ad50, and Pan9 (also known as AdC68)), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus), and poxvirus (e.g., vaccinia, modified vaccinia Ankara (MVA), fowlpox and canarypox). Other viruses useful for delivering polynucleotides encoding a transcription factor or other gene product to a HUCPVC include Norwalk virus, togavirus, flavivirus, reoviruses, papovavirus, hepadnavirus, and hepatitis virus. Adenoviruses and retroviruses (including lentiviruses) are particularly attractive modalities for gene therapy applications, as discussed below, due to the ability to genetically modify and exploit the life cycle of these viruses.

Another means of viral introduction is the utilization of recombinant adenoviral vectors, which offer several significant advantages for the expression of a transcription factor or other gene product in HUCPVCs. The viruses can be prepared at extremely high titer, infect non-replicating cells, and confer high-efficiency and high-level transduction of target cells in vivo after directed injection or perfusion. Furthermore, as adenoviruses do not integrate their DNA into the host genome, this gene therapy modality has a reduced risk of inducing spontaneous proliferative disorders. In animal models, adenoviral gene transfer has generally been found to mediate high-level expression for approximately one week. The duration of transgene expression may be prolonged, and ectopic expression reduced, by using tissue-specific promoters. Other improvements in the molecular engineering of the adenoviral vector itself have produced more sustained transgene expression and less inflammation. This is seen with so-called "second generation" vectors harboring specific mutations in additional early adenoviral genes and "gutless" vectors in which virtually all the viral genes are deleted utilizing a cre-lox. Examples of adenoviruses that can be used as a viral vector include those having, or derived from, the serotypes Ad2, Ad5, Ad11, Ad12, Ad24, Ad26, Ad34, Ad35, Ad40, Ad48, Ad49, Ad50, and Pan9 (also known as AdC68).

Recombinant adeno-associated viruses (rAAV), which are derived from non-pathogenic parvoviruses, can be used to express a donor gene, such as a gene encoding a transcription factor or other gene product, as these vectors evoke almost no cellular immune response, and produce transgene expression lasting months in most systems. The AAV genome is built of single stranded DNA, and includes inverted terminal repeats (ITRs) at both ends of the DNA strand, and two open reading frames: rep and cap, encoding replication and capsid proteins, respectively. A donor gene (e.g., a gene encoding a transcription factor or other gene product) can replace the native rep and cap genes. AAVs can be made with a variety of different serotype capsids which have varying tropism for different tissue types. Examples of AAV serotypes that can be used include but are not limited to AAV1, AAV2, AAV3, AAV4, AAV5, AAV6, AAV7, AAV8, AV9, and AAVrh10. AAV vectors can be produced, for example, by triple transfection of subconfluent HEK293 cells by three plasmids: AAV cis-plasmid containing the donor gene of interest (e.g., a gene encoding a transcription factor or other gene product), AAV trans-plasmid containing AAV rep and cap genes, and an adenovirus helper plasmid, e.g., pDF6. Incorporation of a tissue-specific promoter is, again, typically beneficial.

Another viral vector that can be used to deliver genes into a subject or cells is a retrovirus, including a lentivirus. As opposed to adenoviruses, the genetic material in retroviruses is in the form of RNA molecules, while the genetic material of their hosts is in the form of DNA. When a retrovirus infects a host cell, it will introduce its RNA together with some enzymes into the cell. This RNA molecule from the retrovirus will produce a double-stranded DNA copy (provirus) from its RNA molecules through a process called reverse transcription. Following transport into the cell nucleus, the proviral DNA is integrated in a host chromosome, permanently altering the genome of the infected cell and any progeny cells that may arise. The ability to permanently introduce a gene encoding a polypeptide or oligonucleotide into a cell such as a HUCPVC is the defining characteristic of retroviruses used for gene therapy. Retroviruses include lentiviruses, a family of viruses including human immunodeficiency virus (HIV) that includes several accessory proteins to facilitate viral infection and proviral integration. Additional examples of retroviruses include: avian leukosis-sarcoma, mammalian C-type, B-type viruses, D-type viruses, HTLV-BLV group, and spumavirus.

A retrovirus for gene therapy may be one that is modified to direct the insertion of the donor gene incorporated in the genome of the virus into a non-arbitrary position in the genome of the host, e.g., using a zinc finger nuclease or by including sequences, such as the beta-globin locus control region, to direct the site of integration to specific chromosomal sites. Retroviruses and lentiviruses have considerable utility for gene therapy applications. Current, "third-generation" lentiviral vectors feature total replication incompetence, broad tropism, and increased gene transfer capacity for mammalian cells. Lentiviruses pseudotyped with, e.g., vesicular stomatitis virus glycoprotein (VSV-G) or feline endogenous virus RD114 envelope glycoprotein can be used to transduce HUCPVCs. U.S. Pat. Nos. 5,919,458, 5,994,136, and 7,198,950 describe the production and use of lentiviruses to genetically modify target cells.

Besides adenoviral and retroviral vectors, other viral vectors and techniques are known in the art that can be used to transfer a donor gene encoding a desired polypeptide or oligonucleotide (e.g., a gene encoding a transcription factor or other gene product) into a subject or cells. These viruses include, e.g., poxviruses (e.g., vaccinia virus and modified vaccinia virus Ankara (MVA); see, e.g., U.S. Pat. Nos. 4,603,112 and 5,762,938), herpesviruses, togaviruses

(e.g., Venezuelan Equine Encephalitis virus; see, e.g., U.S. Pat. No. 5,643,576), picornaviruses (e.g., poliovirus; see, e.g., U.S. Pat. No. 5,639,649), and baculoviruses. Other viruses useful for delivering donor genes include papovavirus, hepadnavirus, and hepatitis virus, for example.

Naked DNA or oligonucleotides (e.g., DNA vectors such as plasmids) encoding a transcription factor or other gene product can also be used to genetically modify fibroblasts. This is the simplest method of non-viral transfection. Clinical trials carried out using intramuscular injection of a naked DNA plasmid have had some success; however expression has been low in comparison to other methods of transfection. Other efficient methods for delivery of naked DNA exist such as electroporation and the use of a "gene gun," which shoots DNA-coated gold particles into the cell using high pressure gas.

To improve the delivery of a DNA vector (e.g., a plasmid) into fibroblasts, the DNA can be protected from damage and its entry into the cell facilitated. Lipoplexes and polyplexes have the ability to protect transfer DNA from undesirable degradation during the transfection process. Plasmid DNA can be covered with lipids in an organized structure like a micelle or a liposome. When the organized structure is complexed with DNA it is called a lipoplex. There are three types of lipids, anionic (negatively-charged), neutral, or cationic (positively-charged). Lipoplexes that utilize cationic lipids have proven utility for gene transfer. Cationic lipids, due to their positive charge, naturally complex with the negatively charged DNA. Also as a result of their charge they interact with the cell membrane, endocytosis of the lipoplex occurs, and the DNA is released into the cytoplasm. The cationic lipids also protect against degradation of the DNA by the cell. Complexes of polymers with DNA are called polyplexes. Most polyplexes consist of cationic polymers and their production is regulated by ionic interactions. One large difference between the methods of action of polyplexes and lipoplexes is that polyplexes cannot release their DNA load into the cytoplasm, so to this end, co-transfection with endosome-lytic agents (to lyse the endosome that is made during endocytosis) such as inactivated adenovirus must occur. However, this is not always the case; polymers such as polyethylenimine have their own method of endosome disruption as does chitosan and trimethylchitosan.

In some aspects gene editing is used to genetically modify fibroblasts. Broadly, gene editing approaches are based on precise, targeted changes to the genome of organisms. Gene editing may be used to alter the genome sequence (for example, by incorporation of point mutations, insertions, or deletions). Gene editing approaches can be used to 'knock-in' heterologous nucleic acid sequences into the genome at targeted locations. A variety of gene editing approaches are known in the art, including but not limited to clustered regularly interspaced short palindromic repeats (CRISPR)-Cas (e.g., Cas9) gene editing (see, e.g., U.S. Pat. Nos. 8,697,359 and 8,771,945), transcription activator-like effector based nuclease (TALEN) gene editing, zinc-finger nuclease (ZFN) gene editing, or meganuclease gene editing (see, *e.g.,* U.S. Pat. No. 8,021,867).

### V. Methods of Treatment

Aspects of the disclosure include methods of treating an individual for a medical condition by providing to the individual fibroblasts and/or a product generated by or derived from said fibroblasts, including exosomes and so forth. In particular aspects, the individual has an inflammatory disease, including an autoimmune disease, or is at risk for having an inflammatory disease, including an autoimmune disease as compared to the general population. The individual may be at risk for having one or more risk factors, such as a personal or family history, one or more genetic markers, and so forth. The compositions may be used for *in vivo, in vitro,* or *ex vivo* administration.

The therapy described herein may comprise administration of a combination of therapeutic compositions, such as a first inflammatory disease therapy (e.g., fibroblasts and/or a product generated by or derived from said fibroblasts) and one or more additional inflammatory disease therapies. The therapies may be administered in any suitable manner known in the art. For example, the first and one or more additional inflammatory disease therapies may be administered sequentially (at different times) or concurrently (at the same time or approximately the same time; also "simultaneously" or "substantially simultaneously"). In some aspects, the first and one or more additional inflammatory disease therapies may be administered in a separate composition. In some aspects, the first and one or more additional inflammatory disease therapies may be in the same composition. The different therapies may be administered in one composition or in more than one composition, such as 2 compositions, 3 compositions, or 4 compositions. Various combinations of the agents may be employed.

### A. Carriers

In some aspects, pharmaceutical compositions of the present disclosure comprise an effective amount of one or more compositions comprising fibroblasts and/or a product generated by or derived from said fibroblasts dissolved or dispersed in a pharmaceutically acceptable carrier. The phrases "pharmaceutical" and "pharmacologically acceptable" and used interchangeably herein refer to molecular entities and compositions that do not produce an adverse, allergic or other untoward reaction when administered to a subject, such as, for example, a human, as appropriate, and do not interfere with the therapeutic methods of the disclosure. The preparation of a pharmaceutical composition that comprises fibroblasts and/or a product generated by or derived from said fibroblasts, or additional active ingredient(s), will be known to those of skill in the art in light of the present disclosure, as described in Remington: The Science and Practice of Pharmacy, 21st Ed. Lippincott Williams and Wilkins, 2005. Moreover, for administration to a subject, it will be understood that preparations should meet sterility, pyrogenicity, general safety, and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the pharmaceutical compositions is contemplated. The compositions comprising fibroblasts and/or a product generated by or derived from said fibroblasts may comprise different types of carriers depending on whether it is to be administered in solid, liquid or aerosol form, and whether it needs to be sterile for such routes of administration, such as injection.

Further in accordance with the present disclosure, the composition of the present disclosure suitable for administration may be provided in a pharmaceutically acceptable carrier with or without an inert diluent. The carrier should be assimilable and includes liquid, semi-solid, i.e., pastes, or solid carriers. Except insofar as any conventional media, agent, diluent or carrier is detrimental to the recipient or to the therapeutic effectiveness of a composition contained therein, its use in practicing the methods of the present disclosure is appropriate. Examples of carriers or diluents include fats, oils, water, saline solutions, lipids, liposomes, resins, binders, fillers, alcohols, and the like, or combinations thereof. The composition may also comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

In accordance with the present disclosure, the composition is combined with the carrier in any convenient and practical manner, i.e., by solution, suspension, emulsification, admixture, encapsulation, absorption and the like. Such procedures are routine for those skilled in the art. The compositions comprising fibroblasts and/or a product generated by or derived from said fibroblasts may be lyophilized.

In a specific aspect of the present disclosure, the composition is combined or mixed thoroughly with a semi-solid or solid carrier. The mixing can be carried out in any convenient manner such as grinding. Stabilizing agents can be also added in the mixing process in order to protect the composition from loss of therapeutic activity, i.e., denaturation in the stomach. Examples of stabilizers for use in the composition include buffers, amino acids such as glycine and lysine, carbohydrates such as dextrose, mannose, galactose, fructose, lactose, sucrose, maltose, sorbitol, mannitol, etc.

In further aspects, the present disclosure may include the use of a pharmaceutical lipid vehicle compositions that incorporate compositions comprising fibroblasts and/or a product generated by or derived from said fibroblasts, one or more lipids, and an aqueous solvent. As used herein, the term "lipid" will be defined to include any of a broad range of substances that is characteristically insoluble in water and extractable with an organic solvent. This broad class of compounds is well known to those of skill in the art, and as the term "lipid" is used herein, it is not limited to any particular structure. Examples include compounds which contain long-chain aliphatic hydrocarbons and their derivatives. A lipid may be naturally occurring or synthetic (i.e., designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Also described herein are compounds such as lipids.

One of ordinary skill in the art would be familiar with the range of techniques that can be employed for dispersing a composition in a lipid vehicle. For example, the composition(s) comprising fibroblasts and/or a product generated by or derived from said fibroblasts may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The composition(s) comprising fibroblasts and/or a product generated by or derived from said fibroblasts may be formulated into a composition in a free base, neutral or salt form. Pharmaceutically acceptable salts, include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Upon formulation, solutions will be administered in a manner compatible with the dosage formulation and in such amount as is therapeutically effective. The formulations are easily administered in a variety of dosage forms such as formulated for parenteral administrations such as injectable solutions, or aerosols for delivery to the lungs, or formulated for alimentary administrations such as drug release capsules and the like.

### A. Routes of Administration

The therapeutic agents may be administered by the same route of administration or by different routes of administration. The route of administration of the composition may be, for example, intravenously, intracerebrally, intracranially, intramuscularly, subcutaneously, topically, orally, mucosally, intradermally, transdermally, intraperitoneally, intraarterially, intraorbitally, by implantation, intravaginally, intrarectally, intrathecally, intraarticularly, intraventricularly, intrasynovially, or intranasally; by inhalation, injection, infusion, continuous infusion, localized perfusion bathing target cells directly, via a catheter, via a lavage; in creams or in lipid compositions (e.g., liposomes); by other method or any combination of the forgoing as would be known to one of ordinary skill in the art (see, for example, Remington's Pharmaceutical Sciences, 18th Ed. Mack Printing Company, 1990).

In some aspects, the composition(s) comprising fibroblasts and/or a product generated by or derived from said fibroblasts is delivered systemically or locally. In some aspects, the composition comprising fibroblasts and/or a product generated by or derived from said fibroblasts is delivered by peripheral injection, such as intravenous injection.

### 1. Parenteral Routes

Thus, in some aspects, the composition(s) comprising fibroblasts and/or a product generated by or derived from said fibroblasts may be administered *via* a parenteral route. As used herein, the term "parenteral" includes routes that bypass the alimentary tract. Specifically, the pharmaceutical compositions disclosed herein may be administered for example, but not limited to retro-orbitally, intracerebrally, intracranially, intravenously, intradermally, intramuscularly, intraarterially, intrathecally, subcutaneous, or intraperitoneally U.S. Pat. Nos. 6,7537,514, 6,613,308, 5,466,468, 5,543,158; 5,641,515; and 5,399,363.

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (see, *e.g.,* U.S. Patent 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, polyol (*i.e.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in isotonic NaCl solution and injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions may be prepared by incorporating the active compounds in the required amount in the appropriate solvent with various of the other ingredients enumerated above, as required, followed by filtered sterilization, for example. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, *e.g*., water or a saline solution, with or without a stabilizing agent.

### 2. Alimentary Routes

In particular aspects of the present disclosure, the composition(s) comprising fibroblasts and/or a product generated by or derived from said fibroblasts is formulated to be administered *via* an alimentary route. Alimentary routes include all possible routes of administration in which the composition is in direct contact with the alimentary tract. Specifically, the pharmaceutical compositions disclosed herein may be administered orally, buccally, rectally, or sublingually. As such, these compositions may be formulated with an inert diluent or with an assimilable edible carrier, or they may be enclosed in hard- or soft- shell gelatin capsule, or they may be compressed into tablets, or they may be incorporated directly with the food of the diet.

In certain aspects, the active compounds may be incorporated with excipients and used in the form of ingestible tablets, buccal tables, troches, capsules, elixirs, suspensions, syrups, wafers, and the like (Mathiowitz et al., 1997; Hwang et al., 1998; U.S. Pat. Nos. 5,641,515; 5,580,579 and 5,792, 451). The tablets, troches, pills, capsules and the like may also contain the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, etc. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. When the dosage form is a capsule, it may contain, in addition to materials of the above type, carriers such as a liquid carrier. Gelatin capsules, tablets, or pills may be enterically coated. Enteric coatings prevent denaturation of the composition in the stomach or upper bowel where the pH is acidic. See, e.g., U.S. Pat. No. 5,629,001. Upon reaching the small intestines, the basic pH therein dissolves the coating and permits the composition to be released and absorbed by specialized cells, e.g., epithelial enterocytes and Peyer's patch M cells. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

For oral administration, the composition(s) comprising fibroblasts and/or a product generated by or derived from said fibroblasts of the present disclosure may alternatively be incorporated with one or more excipients in the form of a mouthwash, dentifrice, buccal tablet, oral spray, or sublingual orally- administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically- effective amount to a composition that may include water, binders, abrasives, flavoring agents, foaming agents, and humectants. Alternatively, the compositions may be fashioned into a tablet or solution form that may be placed under the tongue or otherwise dissolved in the mouth.

Additional formulations that are suitable for other modes of alimentary administration include suppositories. Suppositories are solid dosage forms of various weights and shapes, usually medicated, for insertion into the rectum. After insertion, suppositories soften, melt or dissolve in the cavity fluids. In general, for suppositories, traditional carriers may include, for example, polyalkylene glycols, triglycerides or combinations thereof. In certain aspects, suppositories may be formed from mixtures containing, for example, the active ingredient in the range of about 0.5% to about 10% (by weight), and preferably about 1% to about 2% (by weight).

### 3. Miscellaneous Routes

In other aspects of the disclosure, the composition(s) comprising fibroblasts and/or a product generated by or derived from said fibroblasts may be formulated for administration *via* various miscellaneous routes, for example, topical (i.e., transdermal) administration, mucosal administration (intranasal, vaginal, etc.) and/or inhalation.

Pharmaceutical compositions for topical administration may include the active compound formulated for a medicated application such as an ointment, paste, cream or powder. Ointments include all oleaginous, adsorption, emulsion and water-soluble based compositions for topical application, while creams and lotions are those compositions that include an emulsion base only. Topically administered medications may contain a penetration enhancer to facilitate adsorption of the active ingredients through the skin. Suitable penetration enhancers include glycerin, alcohols, alkyl methyl sulfoxides, pyrrolidones and luarocapram. Possible bases for compositions for topical application include polyethylene glycol, lanolin, cold cream and petrolatum as well as any other suitable absorption, emulsion or water-soluble ointment base. Topical preparations may also include emulsifiers, gelling agents, and antimicrobial preservatives as necessary to preserve the active ingredient and provide for a homogenous mixture. Transdermal administration of the present disclosure may also comprise the use of a "patch". For example, the patch may supply one or more active substances at a predetermined rate and in a continuous manner over a fixed period of time.

In certain aspects, the pharmaceutical composition(s) comprising fibroblasts and/or a product generated by or derived from said fibroblasts may be delivered by eye drops, intranasal sprays, inhalation, and/or other aerosol delivery vehicles. Methods for delivering compositions directly to the lungs via nasal aerosol sprays has been described e.g., in U.S. Pat. Nos. 5,756,353 and 5,804,212. Likewise, the delivery of drugs using intranasal microparticle resins (see, e.g., Takenaga et al., 1998) and lysophosphatidyl-glycerol compounds (see, e.g., U.S. Pat. No. 5,725, 871) are also well-known in the pharmaceutical arts. Likewise, transmucosal drug delivery in the form of a polytetrafluoroetheylene support matrix is described in, e.g., U.S. Pat. No. 5,780,045.

The term aerosol refers to a colloidal system of finely divided solid of liquid particles dispersed in a liquefied or pressurized gas propellant. The typical aerosol of the present disclosure for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### B. Dosing

The appropriate dosage amount of a composition(s) of the present disclosure administered to the subject can be determined by physical and physiological factors such as body weight, severity and course of condition, the type of disease being treated, the clinical condition of the individual, previous or concurrent therapeutic interventions, the individual's clinical history and response to the treatment, idiopathy of the subject, the route of administration, and the discretion of the attending physician. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain aspects, pharmaceutical compositions may comprise, for example, at least about 0.1% (by weight) of an active compound. In other aspects, the active compound may comprise between about 2% to about 75% of the weight of the unit, or between about 25% to about 60%, for example, and any range derivable therein. Naturally, the amount of active compound(s) in each therapeutically useful composition may be prepared in such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

The treatments may include various "unit doses." Unit dose is defined as containing a predetermined-quantity of the therapeutic composition. The quantity to be administered, and the particular route and formulation, is within the skill of determination of those in the clinical arts. A unit dose need not be administered as a single injection but may comprise continuous infusion over a set period of time. In some aspects, a unit dose comprises a single administrable dose.

The quantity to be administered, both according to number of treatments and unit dose, depends on the treatment effect desired. An effective dose is understood to refer to an amount necessary to achieve a particular effect. Furthermore, such doses can be administered at multiple times during a day, and/or on multiple days, weeks, or months.

In some aspects, fibroblasts are administered between 1000 cells per kilogram body weight and 3 million cells per kilogram body weight, such as 1 million cells per kilogram body weight. In specific aspects, fibroblasts are administered between 1000 cells and 2 million cells, 1000 cells and 1 million cells, 1000 cells and 500,000 cells, 1000 cells and 250,000 cells, 1000 cells and 150,000 cells, 1000 cells and 100,000 cells, or 1000 cells and 10,000 cells per kilogram per body weight.

Precise amounts of the therapeutic composition also depend on the judgment of the practitioner and are peculiar to each individual. Factors affecting dose include physical and clinical state of the patient, the route of administration, the intended goal of treatment (alleviation of symptoms versus cure) and the potency, stability and toxicity of the particular therapeutic substance or other therapies a subject may be undergoing.

### VI. Kits of the Disclosure

Any of the cellular and/or non-cellular compositions described herein or similar thereto may be comprised in a kit. In a non-limiting example, one or more reagents for use in methods for treatment of inflammation may be comprised in a kit. Such reagents may include fibroblasts, derivatives thereof, media, enzymes, buffers, nucleotides, salts, primers, and so forth. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, or may be a substrate with multiple compartments for a desired reaction.

Some components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile acceptable buffer and/or other diluent.

In specific aspects, reagents and materials include primers for amplifying desired sequences, nucleotides, suitable buffers or buffer reagents, salt, and so forth, and in some cases the reagents include apparatus or reagents for isolation of a particular desired cell(s).

In particular aspects, there are one or more apparatuses in the kit suitable for extracting one or more samples from an individual. The apparatus may be a syringe, fine needles, scalpel, and so forth.

### EXAMPLES

### EXAMPLE 1

### SUPPRESSION OF IL-17 PRODUCTION BY CULTURE OF FIBROBLASTS WITH CD4 CELLS PRIMED BY IL-6

Human dermal fibroblasts were obtained from American Type Culture Collection and cultured in the presence of 1 µM oxytocin (to activate the fibroblasts to suppress IL-17 production) for 48 hours. Fibroblast cells were added to a culture of CD4 T cells incubated with the indicated concentrations of IL-6 (to stimulate production of IL17). CD4 cells were cultured at 80,000 cells per well. Cultures of CD4 cells and fibroblasts were maintained for 48 hours. IL-17 concentration was assessed by ELISA (FIG. 1). The order of bars on the figure from left to right are no fibroblast control, 10,000 fibroblasts, and 20,000 fibroblasts. Fibroblast co-culture profoundly inhibited IL-17 production.

### EXAMPLE 2

### SUPPRESSION OF IL-17 PRODUCTION BY CULTURE OF FIBROBLASTS WITH CD4 CELLS PRIMED BY IL-6

Mice having collagen-induced arthritis (CIA) after a second injection of collagen as described in [3] were injected with 100,000 cells of CD73-positive fibroblasts, bone marrow mesenchymal stem cells (MSCs), or saline. One group of mice were not induced to undergo collagen-induced arthritis (control). Plasma IL-17 was assessed at days 7, 14 and 21 (FIG. 2). The order of bars on the figure from left to right are no CIA control, CIA and saline, CIA and MSCs, and CIA and CD73-positive fibroblasts. Fibroblast co-culture profoundly inhibited IL-17 production.

### REFERENCES

1. Yao, Z., et al., Human IL-17: a novel cytokine derived from T cells. J Immunol, 1995. 155(12): p. 5483-6.
2. Fossiez, F., et al., T cell interleukin-17 induces stromal cells to produce proinflammatory and hematopoietic cytokines. J Exp Med, 1996. 183(6): p. 2593-603.
3. Zheng, X., et al., Treatment of autoimmune arthritis using RNA interference-modulated dendritic cells. J Immunol, 2010. 184(11): p. 6457-64.

## Claims

1. Fibroblasts for use in a method of treating inflammation in an individual, the method comprising the step of providing to the individual an effective amount of said fibroblasts, wherein said fibroblasts express interleukin-3 receptor, CD73 and CD56.

2. Fibroblasts for use according to claim 1, wherein prior to the providing step, the fibroblasts are exposed to one or more agents and/or conditions that activate the fibroblasts to suppress IL-17 production, wherein said one or more agents and/or conditions that activate the fibroblasts to suppress IL-17 production comprises oxytocin.

3. Fibroblasts for use according to claim 1, wherein prior to the providing step, the fibroblasts are exposed to one or more agents and/or conditions that activate NF-kappa B in the fibroblasts, wherein the one or more agents and/or conditions are selected from the group consisting of hydrogen peroxide, ozone, TNF-alpha, IL-1, osmotic shock, mechanical agitation, and a combination thereof.

4. Fibroblasts for use according to claim 3, wherein:
(a) prior to the providing step, the fibroblasts are exposed to 0.1 µM to 10 µM of oxytocin; and/or
(b) prior to the providing step, the fibroblasts are exposed to oxytocin for 1 minute to 96 hours; and/or
(c) prior to the providing step, the fibroblasts are exposed to 1µM of oxytocin for 48 hours.

5. Fibroblasts for use according to claim 3, wherein NF-kappa B-activated fibroblasts produce one or more of IL-10, IL-35, and IL-37.

6. Fibroblasts for use according to any one of claims 1-5, wherein the providing step comprises contacting the fibroblasts with IL17-producing cells *in vivo.*

7. Fibroblasts for use according to claim 6, wherein the IL17-producing cells are immunological cells, optionally wherein the immunological cells are selected from the group consisting of i) monocytes; ii) T cells; iii) Th17 cells; iv) neutrophils; v) stromal cells; vi) mesenchymal stem cells; vii) dendritic cells; and viii) a combination thereof.

8. Fibroblasts for use according to any one of claims 1-7, wherein:
(a) the fibroblasts are obtained from a group of placental, fetal, neonatal and/or adult tissues selected from the group consisting of placenta; amniotic fluid, umbilical cord; umbilical cord blood; embryonic fibroblasts; mobilized peripheral blood; omentum; hair follicle; ear lobe skin; skin; bone marrow; foreskin; adipose tissue; Wharton's Jelly; plastic surgery related by-product; nail matrix; heart; blood vessels; skeletal muscle; liver; pancreas; brain; and a combination thereof, optionally wherein the mobilized peripheral blood is obtained by administration to the individual of agents selected from the group consisting of VLA-5 antibodies; G-CSF; M-CSF; GM-CSF; FLT-3L; TNF-alpha; EGF; FGF-1; FGF-2; FGF-5; VEGF; and a combination thereof; and/or
(b) said fibroblasts are autologous, or allogeneic with respect to the individual; and/or
(c) said fibroblasts are xenogeneic with respect to the individual.

9. Fibroblasts for use according to any one of claims 1-8, wherein the fibroblasts express markers selected from the group consisting of CD90 (Thy-1) and collagen.

10. Fibroblasts for use according to any one of claims 1-9, wherein the fibroblasts are provided to the individual intravenously by injection.

11. Fibroblasts for use according to any one of claims 1-10, wherein the individual has an inflammatory disease.

12. Fibroblasts for use according to any one of claims 1-11, wherein the individual has atherosclerosis, cancer, Graft-versus-host disease (GvHD), cystic fibrosis, depression, rheumatoid arthritis, psoriasis, multiple sclerosis, inflammatory bowel diseases, rheumatoid arthritis (RA), multiple sclerosis (MS), psoriasis, Crohn's disease, systemic lupus erythematosus (SLE), asthma, Behçet's disease, hyper IgE syndrome, or a combination thereof.

13. Fibroblasts for use according to any one of claims 1-12, wherein the individual has arthritis.

14. Fibroblasts for use according to any one of claims 1-12, wherein the individual has colitis.

## Patentansprüche

1. Fibroblasten zur Verwendung in einem Verfahren des Behandelns von Entzündung in einem Individuum, wobei das Verfahren den Schritt des Bereitstellens einer wirksamen Menge der Fibroblasten an das Individuum umfasst, wobei die Fibroblasten Interleukin-3-Rezeptor, CD73 und CD56 exprimieren.

2. Fibroblasten zur Verwendung nach Anspruch 1, wobei vor dem Bereitstellungsschritt die Fibroblasten einem oder mehreren Mitteln und/oder Zuständen ausgesetzt sind, die die Fibroblasten aktivieren, um IL-17-Produktion zu unterdrücken, wobei das eine oder mehrere Mittel und/oder die Zustände, die die Fibroblasten aktivieren, um IL-17-Produktion zu unterdrücken, Oxytocin umfasst.

3. Fibroblasten zur Verwendung nach Anspruch 1, wobei vor dem Bereitstellungsschritt die Fibroblasten einem oder mehreren Mitteln und/oder Zuständen ausgesetzt sind, die NF-Kappa-B in den Fibroblasten aktivieren, wobei das eine oder mehrere Mittel und/oder die Zustände ausgewählt sind aus der Gruppe bestehend aus Wasserstoffperoxid, Ozon, TNF-Alpha, IL-1, osmotischem Schock, mechanischer Bewegung und einer Kombination davon.

4. Fibroblasten zur Verwendung nach Anspruch 3, wobei:
(a) vor dem Bereitstellungsschritt die Fibroblasten 0,1 µM bis 10 µM Oxytocin ausgesetzt sind; und/oder
(b) vor dem Bereitstellungsschritt die Fibroblasten Oxytocin 1 Minute bis 98 Stunden lang ausgesetzt sind; und/oder
(c) vor dem Bereitstellungsschritt die Fibroblasten 1 µM Oxytocin 48 Stunden lang ausgesetzt sind.

5. Fibroblasten zur Verwendung nach Anspruch 3, wobei NF-Kappa-B-aktivierte Fibroblasten ein oder mehrere aus IL-10, IL-35 und IL-37 produzieren.

6. Fibroblasten zur Verwendung nach einem der Ansprüche 1-5, wobei der Bereitstellungsschritt das Kontaktieren der Fibroblasten mit IL-17-produzierenden Zellen *in vivo* umfasst.

7. Fibroblasten zur Verwendung nach Anspruch 6, wobei die IL-17-produzierenden Zellen immunologische Zellen sind, wobei optional die immunologischen Zellen ausgewählt sind aus der Gruppe bestehend aus i) Monocyten; ii) T-Zellen; iii) Th17-Zellen; iv) Neutrophilen; v) Stromazellen; vi) mesenchymalen Stammzellen; vii) dendritischen Zellen; und viii) einer Kombination davon.

8. Fibroblasten zur Verwendung nach einem der Ansprüche 1-7, wobei:
(a) die Fibroblasten aus einer Gruppe aus Plazenta-, neonatalem und/oder adultem Gewebe erhalten werden, ausgewählt aus der Gruppe bestehend aus Plazenta; Fruchtwasser, Nabelschnur; Nabelschnurblut; embryonalen Fibroblasten; mobilisiertem peripherem Blut; Omentum; Haarfollikel; Ohrläppchenhaut; Haut; Knochenmark; Vorhaut; Fettgewebe; Wharton-Sulze; Nebenprodukt in Verbindung mit plastischer Chirurgie; Nagelmatrix; Herz; Blutgefäßen; Skelettmuskel; Leber; Pankreas; Gehirn; und einer Kombination davon, wobei optional das mobilisierte periphere Blut erhalten wird durch Verabreichung von Mitteln, ausgewählt aus der Gruppe bestehend aus VLA-5-Antikörpern; G-CSF; M-CSF; GM-CSF; FLT-3L; TNF-Alpha; EGF; FGF-1; FGF-2; FGF-5; VEGF; und einer Kombination davon, an das Individuum; und/oder
(b) die Fibroblasten autolog oder allogen in Bezug auf das Individuum sind; und/oder
(c) die Fibroblasten xenogen in Bezug auf das Individuum sind.

9. Fibroblasten zur Verwendung nach einem der Ansprüche 1-8, wobei die Fibroblasten Marker exprimieren, ausgewählt aus der Gruppe bestehend aus CD90 (Thy-1) und Kollagen.

10. Fibroblasten zur Verwendung nach einem der Ansprüche 1-9, wobei die Fibroblasten dem Individuum intravenös durch Injektion bereitgestellt werden.

11. Fibroblasten zur Verwendung nach einem der Ansprüche 1-10, wobei das Individuum eine Entzündungskrankheit hat.

12. Fibroblasten zur Verwendung nach einem der Ansprüche 1-11, wobei das Individuum Atherosklerose, Krebs, Graft-versus-host-Krankheit (GvHD), zystische Fibrose, Depression, rheumatoide Arthritis, Psoriasis, Multiple Sklerose, entzündliche Darmerkrankungen, rheumatoide Arthritis (RA), Multiple Sklerose (MS), Psoriasis, Morbus Crohn, systemischen Lupus erythematodes (SLE), Asthma, Behçet-Krankheit, Hyper-IgE-Syndrom oder eine Kombination davon hat.

13. Fibroblasten zur Verwendung nach einem der Ansprüche 1-12, wobei das Individuum Arthritis hat.

14. Fibroblasten zur Verwendung nach einem der Ansprüche 1-12, wobei das Individuum Colitis hat.

## Revendications

1. Fibroblastes destinés à être utilisés dans un procédé de traitement d'une inflammation chez un individu, le procédé comprenant l'étape consistant à fournir à l'individu une quantité efficace desdits fibroblastes, dans lesquels lesdits fibroblastes expriment un récepteur d'interleukine-3, CD73 et CD56.

2. Fibroblastes destinés à être utilisés selon la revendication 1, dans lesquels, avant l'étape de fourniture, les fibroblastes sont exposés à un ou plusieurs agents et/ou conditions qui activent les fibroblastes afin de supprimer la production d'IL-17, dans lesquels ledit ou lesdits agents et/ou conditions qui activent les fibroblastes afin de supprimer la production d'IL-17 comprennent l'ocytocine.

3. Fibroblastes destinés à être utilisés selon la revendication 1, dans lesquels, avant l'étape de fourniture, les fibroblastes sont exposés à un ou plusieurs agents et/ou conditions qui activent NF-kappa B dans les fibroblastes, dans lesquels le ou les agents et/ou conditions sont choisis dans le groupe constitué par le peroxyde d'hydrogène, l'ozone, TNF-alpha, IL-1, un choc osmotique, une agitation mécanique, et une combinaison de ceux-ci.

4. Fibroblastes destinés à être utilisés selon la revendication 3, dans lesquels :
(a) avant l'étape de fourniture, les fibroblastes sont exposés à 0,1 µM à 10 µM d'ocytocine ; et/ou
(b) avant l'étape de fourniture, les fibroblastes sont exposés à l'ocytocine pendant 1 minute à 96 heures ; et/ou
(c) avant l'étape de fourniture, les fibroblastes sont exposés à 1 µM d'ocytocine pendant 48 heures.

5. Fibroblastes destinés à être utilisés selon la revendication 3, dans lesquels des fibroblastes activés par NF-kappa B produisent une ou plusieurs parmi IL-10, IL-35, et IL-37.

6. Fibroblastes destinés à être utilisés selon l'une quelconque des revendications 1 à 5, dans lesquels l'étape de fourniture comprend la mise en contact des fibroblastes avec des cellules productrices d'IL-17 *in vivo.*

7. Fibroblastes destinés à être utilisés selon la revendication 6, dans lesquels les cellules productrices d'IL-17 sont des cellules immunologiques, éventuellement dans lesquels les cellules immunologiques sont choisies dans le groupe constitué par i) des monocytes ; ii) des lymphocytes T ; iii) des cellules Th17 ; iv) des neutrophiles ; v) des cellules stromales ; vi) des cellules souches mésenchymateuses ; vii) des cellules dendritiques ; et viii) une combinaison de ceux-ci.

8. Fibroblastes destinés à être utilisés selon l'une quelconque des revendications 1 à 7, dans lesquels :
(a) les fibroblastes sont obtenus à partir d'un groupe de tissus placentaires, fœtaux, néonatals et/ou adultes choisis dans le groupe constitué par le placenta ; le liquide amniotique, le cordon ombilical ; le sang du cordon ombilical ; des fibroblastes embryonnaires ; le sang périphérique mobilisé ; l'omentum ; un follicule pileux ; la peau du lobe de l'oreille ; la peau ; la moelle osseuse ; le prépuce ; le tissu adipeux ; la gelée de Wharton ; un sous-produit lié à la chirurgie plastique ; la matrice de l'ongle ; le cœur ; les vaisseaux sanguins ; un muscle squelettique ; le foie ; le pancréas ; le cerveau ; et une combinaison de ceux-ci, éventuellement dans lesquels le sang périphérique mobilisé est obtenu par administration à l'individu d'agents choisis dans le groupe constitué par des anticorps VLA-5 ; G-CSF ; M-CSF ; GM-CSF ; FLT-3L ; TNF-alpha ; EGF; FGF-1 ; FGF-2 ; FGF-5 ; VEGF ; et une combinaison de ceux-ci ; et/ou
(b) lesdits fibroblastes sont autologues, ou allogéniques par rapport à l'individu ; et/ou
(c) lesdits fibroblastes sont xénogéniques par rapport à l'individu.

9. Fibroblastes destinés à être utilisés selon l'une quelconque des revendications 1 à 8, dans lesquels les fibroblastes expriment des marqueurs choisis dans le groupe constitué par CD90 (Thy-1) et le collagène.

10. Fibroblastes destinés à être utilisés selon l'une quelconque des revendications 1 à 9, dans lesquels les fibroblastes sont fournis à l'individu par injection intraveineuse.

11. Fibroblastes destinés à être utilisés selon l'une quelconque des revendications 1 à 10, dans lesquels l'individu souffre d'une maladie inflammatoire.

12. Fibroblastes destinés à être utilisés selon l'une quelconque des revendications 1 à 11, dans lesquels l'individu souffre d'athérosclérose, de cancer, de maladie du greffon contre l'hôte (GvHD), de fibrose kystique, de dépression, de polyarthrite rhumatoïde, de psoriasis, de sclérose en plaques, de maladies inflammatoires de l'intestin, de polyarthrite rhumatoïde (PR), de sclérose en plaques (SEP), de psoriasis, de maladie de Crohn, de lupus érythémateux disséminé (LED), d'asthme, de maladie de Behçet, de syndrome hyper-IgE, ou d'une combinaison de ceux-ci.

13. Fibroblastes destinés à être utilisés selon l'une quelconque des revendications 1 à 12, dans lesquels l'individu souffre d'arthrite.

14. Fibroblastes destinés à être utilisés selon l'une quelconque des revendications 1 à 12, dans lesquels l'individu souffre de colite.
